(19) **Europäisches Patentamt**

**European Patent Office**

**Office européen des brevets**

(11) **EP 0 745 372 B2**

(12) **NOUVEAU FASCICULE DE BREVET EUROPEEN**

(45) Date de publication et mention de la décision concernant l'opposition:
**29.05.2002 Bulletin 2002/22**

(45) Mention de la délivrance du brevet:
**11.03.1998 Bulletin 1998/11**

(21) Numéro de dépôt: **96401025.0**

(22) Date de dépôt: **10.05.1996**

(51) Int Cl.⁷: **A61K 7/04**, A61K 7/06,
A61K 7/043, A61K 7/032,
A61K 7/48

(54) **Utilisation d'acide silicique colloidal pour renforcer les matières kératiniques**

Verwendung von kolloidaler Kieselsäure zur Verstärkung von keratinischen Fasern

Use of colloidal silicic acid to reinforce keratineous fibres

(84) Etats contractants désignés:
**DE ES FR GB IT**

(30) Priorité: **30.05.1995 FR 9506386**
**30.05.1995 FR 9506388**
**30.05.1995 FR 9506389**

(43) Date de publication de la demande:
**04.12.1996 Bulletin 1996/49**

(73) Titulaire: **L'OREAL**
**75008 Paris (FR)**

(72) Inventeur: **Ramin, Roland**
**91760 Itteville (FR)**

(74) Mandataire: **Kromer, Christophe**
**L'OREAL - D.P.I.,**
**6, rue Bertrand Sincholle**
**92585 Clichy Cedex (FR)**

(56) Documents cités:
**EP-A- 0 453 628**       **EP-A- 0 504 754**
**EP-A- 0 529 396**       **EP-A- 0 635 260**
**WO-A-94/28877**         **WO-A-95/21124**
**DE-A- 2 053 505**       **DE-A- 2 205 461**
**DE-A- 3 206 448**       **FR-A- 2 578 741**
**US-A- 3 639 572**       **US-A- 4 983 418**
**US-A- 5 071 639**

- **CHEMICAL ABSTRACTS, vol. 89, no. 2, 10 Juillet 1978 Columbus, Ohio, US; abstract no. 11975k, GAYAN MARTINEZ: "NONSETTLING NAIL POLISH COMPOSITION" page 358; XP002013841**
- **M. Pahlow, Das grosse Buch der Heilpflanzen, S. 25, Gräfer und Unzer, 1979**
- **The Journal of Int. Medical Research, 21, 209-215, 1993**
- **M.G. Voronkov et al, Silicium und Leben, Akademie-Verlag, Berlin, S. 123-125 (1975)**

EP 0 745 372 B2

## Description

La présente invention a trait à l'utilisation d'acide silicique colloïdal comme agent renforçateur dans une composition cosmétique à appliquer sur les ongles. Cette composition peut se présenter sous la forme d'une base ou d'un vernis à ongles, d'une base de soin des ongles en milieu huileux.

L'acide silicique colloïdal est fréquemment employé dans des compositions cosmétiques. Le document WO 94/28877 divulgue l'utilisation d'acide silicique colloïdal en très petites quantités (1-500 ppm) dans des compositions aqueuses pour favoriser la pénétration de l'eau, des huiles, du collagène dans l'épiderme et de produits colorants dans les cheveux.

On connaît des compositions à appliquer par exemple sur l'ongle, de type vernis à ongles ou base de soin pour ongles en milieu solvant, comprenant de manière usuelle, au moins un polymère filmogène, éventuellement un agent plastifiant, des pigments, des agents rhéologiques et des solvants.

Une telle composition peut ainsi permettre d'embellir l'ongle, sans toutefois le traiter. Il serait ainsi intéressant de disposer d'une composition cosmétique qui, d'une part, pourrait embellir l'ongle en y déposant un film cosmétiquement acceptable, et qui, d'autre part, serait susceptible d'en renforcer les matières kératiniques, grâce à la présence d'un actif adéquat, appelé dans la suite de la présente description "agent renforçateur".

Les vernis à ongles aqueux sont généralement constitués d'une dispersion aqueuse de particules de polymère filmogène, à laquelle on peut ajouter des cosolvants, des pigments et/ou des colorants, ainsi que des agents rhéologiques. On obtient ainsi des vernis de viscosité adéquate, qui sont applicables aisément sur l'ongle, en une quantité suffisante, et qui présentent une bonne thixotropie. Il peut toutefois être nécessaire de disposer d'une composition susceptible de renforcer les matières kératiniques, par exemple des ongles, des cils ou des cheveux, et qui donc contiendrait un actif adéquat, appelé dans la suite de la présente description "agent renforçateur".

On connaît des bases de soin à appliquer notamment sur l'ongle, se présentant sous la forme d'une huile ou d'un mélange d'huiles, pouvant contenir des additifs usuels, tels que des colorants et/ou des actifs, de manière à traiter, nourrir, soigner et/ou embellir l'ongle.

Il subsiste toutefois le besoin d'une base en milieu huileux, comprenant un actif susceptible de renforcer les ongles, appelé dans la suite de la présente description "agent renforçateur".

La fonction d'un tel agent renforçateur est de permettre, de diminuer la fragilisation des ongles affaiblis, et notamment striés, fendus, mous ou souples, et ayant tendance à se dédoubler.

La demanderesse a constaté que, de manière surprenante, l'acide silicique colloïdal pouvait être utilisé de manière satisfaisante en tant qu'un tel agent renforçateur des ongles.

La présente invention a pour objet l'utilisation de l'acide silice colloïdal en tant qu'agent renforçateur des ongles selon les revendications 1 à 4.

La présente invention a pour premier objet l'utilisation telle que décrite précédemment dans une composition comprenant au moins une matière filmogène et au moins un solvant.

Le solvant peut être un solvant organique et/ou de l'eau. Dans ce dernier cas, la matière filmogène est de préférence présente sous la forme de particules de polymère filmogène en dispersion dans le milieu aqueux.

Un autre objet de l'invention est l'utilisation telle que décrite précédemment dans une composition comprenant un milieu huileux.

L'utilisation d'une composition comprenant de l'acide silicique colloïdal, sur les ongles, permet l'obtention d'ongles plus durs et plus résistants, donc moins cassants. Ce renforcement de la kératine de l'ongle permet également d'obtenir des ongles qui ne se dédoublent plus et/ou qui ne se fendillent plus.

Lorsque la composition comprenant de l'acide silicique colloïdal est une composition dans un solvant organique, elle comprend au moins une matière filmogène qui peut être choisie, notamment, parmi les résines alkydes, acryliques et/ou vinyliques, les polyuréthannes et les polyesters, les celluloses et dérivés cellulosiques telles que la nitrocellulose, et les résines résultant de la condensation de formaldéhyde avec une arylsulfonamide, et leurs mélanges.

La matière filmogène est généralement en solution, par exemple à 5-25% en poids, dans un solvant organique tel que le toluène, le xylène, l'acétate d'éthyle et/ou de butyle, les cétones, les éthers de glycol, les esters et les alcools tels que l'éthanol, l'isopropanol ou le butanol, et leurs mélanges.

La composition peut également comprendre un agent plastifiant et éventuellement des agents rhéologiques.

Parmi les agents plastifiants, on peut citer les citrates, les phtalates, les esters et/ou le camphre, généralement utilisés en une quantité de 5-30% en poids par rapport au poids de la composition.

Parmi les agents rhéologiques, on peut citer les bentonites organophiles, les dérivés de cellulose, les dérivés d'acide polyacrylique réticulé, les gommes de guar ou de caroube, ainsi que les gommes de xanthane.

La composition comprenant de l'acide silicique colloïdal peut également comprendre des particules de polymère filmogène en dispersion dans un milieu aqueux.

Parmi les polymères filmogènes susceptibles d'être utilisés, on peut citer les polyuréthannes, par exemple anioniques, les polyester-polyuréthannes, les polyéther-polyuréthannes, les polymères radicalaires notamment de type acrylique, acrylique styrène et/ou vinylique, les polyesters, les résines alkydes, seuls ou en mélange.

La dispersion peut également comprendre un polymère associatif de type polyuréthanne ou une gomme naturelle telle que la gomme xanthane.

La dispersion présente de préférence un taux de matière sèche de 30-45% en poids.

La composition comprenant de l'acide silicique colloïdal peut également comprendre un milieu huileux.

De préférence elle se présente alors sous la forme d'une base en milieu huileux. Ledit milieu huileux peut comprendre une ou plusieurs huiles, volatiles et/ou non volatiles, par exemple d'origine végétale, minérale, animale et/ou de synthèse, parmi lesquelles on peut citer :

. les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat, les huiles de poisson, le tricaprocaprylate de glycérol, ou les huiles végétales ou animales de formule $R_9COCR_{10}$ dans laquelle $R_9$ représente le reste d'un acide gras supérieur comportant de 7 à 19 atomes de carbone et $R_{10}$ représente une chaîne hydrocarbonée ramifée contenant de 3 à 20 atomes de carbone, par exemple, l'huile de Purcellin ;

. les huiles essentielles naturelles ou synthétiques telles que, par exemple, les huiles d'eucalyptus, de lavandin, de lavande, de vétiver, de citron, de santal, de romarin, de camomille, de sarriette, de noix de muscade, de cannelle, d'hysope, de carvi, d'orange, de géraniol, de cade et de bergamote ;

. les hydrocarbures, tels que l'hexadécane et l'huile de paraffine ;

. les esters d'acide minéral et d'un alcool ;

. les éthers et les polyéthers ;

. les huiles et gommes de silicones.

Dans un mode de réalisation particulier, ledit milieu huileux peut également comprendre une phase aqueuse, avec laquelle il peut former une solution, ou une émulsion huile-dans-eau, eau-dans-huile, voire une émulsion multiple.

L'acide silicique colloïdal susceptible d'être utilisé selon l'invention est une silice pyrogénée ou traitée en surface, qui peut se présenter sous forme de silice pyrogénée hydrophile, de silice pyrogénée hydrophobe, ou de silice traitée en surface par un traitement organique.

Les silices pyrogénées peuvent être obtenues par hydrolyse à haute température d'un composé volatil du silicium dans une flamme oxhydrique. Il s'ensuit la production d'une silice finement divisée. Par réaction, on peut modifier chimiquement la surface de ladite silice, par réduction du nombre de groupes silanols afin d'obtenir une silice hydrophobe.

L'acide silicique colloïdal présente de préférence une taille des particules pouvant être nanométrique à micrométrique, par exemple de l'ordre de 10-200 nm.

L'acide silicique colloïdal selon l'invention peut être entre autre choisi parmi les composés vendus par DEGUSSA, sous les marques Aerosil 200, Aerosil 300 ou Aerosil 380, qui sont des silices hydrophiles, Aerosil MOX80 ou Aerosil COK84 qui sont des silices spéciales, Aerosil R972 qui est une silice hydrophobe, ou encore Aerosil OK412 qui est une silice traitée en surface.

Lorsque la composition est un milieu solvant organique ou aqueux, elle peut comprendre l'acide silicique colloïdal en une quantité de 0,1% à 5% en poids, de préférence en une quantité de 1-2% en poids par rapport au poids total de la composition.

Lorsque la composition est en milieu huileux, elle peut comprendre l'acide silicique colloïdal en une quantité de 0,01 à 10% en poids, de préférence 0,3-3% en poids, par rapport au poids total de la composition.

Les compositions comprenant de l'acide silicique colloïdal peuvent comprendre tout additif connu de l'homme du métier comme étant susceptible d'être incorporé dans une telle composition, tels que des agents d'étalement, des agents mouillants, des agents dispersants, des anti-mousses, des conservateurs, des filtres UV, des colorants, des pigments, des nacres, des actifs tels que le N-butylformal, le D-panthénol, le phytantriol, les vitamines et leurs dérivés, la kératine et ses dérivés, la mélanine, le collagène, la cystine, des parfums, le chitosane et ses dérivés, les céramides, la biotine, les oligo-éléments, la glycérine, les hydrolysats de protéines, les phospholipides, les agents hydratants, ainsi que des cires animales, végétales, minérales, fossiles ou de synthèse, telles que la cire d'abeilles, de caroube, de Candellila, l'ozokérite, les cires microcristallines, les cires et résines de silicone. Bien entendu l'homme du métier veillera à choisir ce ou ces éventuels composés complémentaires, et/ou leur quantité, de manière telle que les propriétés avantageuses de la composition ne soient pas, ou substantiellement pas, altérées par l'adjonction envisagée.

La composition comprenant de l'acide silicique colloïdal peut être préparée par l'homme du métier sur base de ses connaissances générales et selon l'état de la technique.

La composition comprenant de l'acide silicique colloïdal peut se présenter sous la forme d'un produit à appliquer sur les ongles, tel qu'un vernis, une base ou une base de soin.

Les compositions comprenant de l'acide silicique colloïdal peuvent donc se présenter sous la forme d'une base huileuse ou d'une émulsion, et peuvent être utilisées afin de traiter les ongles.

Les compositions peuvent donc être notamment utilisées dans ou en tant que base de soin pour ongles.

L'invention est illustrée plus en détail dans les exemples suivants.

Exemple 1

On prépare un vernis à ongles ayant la composition suivante (% en poids) :

| | |
|---|---|
| . nitrocellulose | 15% |
| . plastifiant et résine | 15% |
| . acide silicique colloïdal (commercialisé sous la marque AEROSIL 200 de Degussa) | 1% |
| . solvant (acétates d'éthyle et de butyle) | qsp 100% |

On obtient un vernis de texture adéquate, qui s'applique aisément sur l'ongle. Il permet, après séchage, l'obtention d'un film lisse et homogène.

Exemple 2

On compare le vernis de l'exemple 1 avec un vernis-témoin de composition :

| | |
|---|---|
| . nitrocellulose | 15% |
| . plastifiant et résine | 15% |
| . solvant (acétates d'éthyle et de butyle) | qsp 100% |

On applique cette composition pendant 8 semaines sur des ongles fragilisés (striés, fendus, mous ou souples, et ayant tendance à se dédoubler).

On l'applique sur tous les ongles d'une seule main, la composition témoin étant appliquée sur l'autre main.

On observe les résultats suivants, obtenus par auto-évaluation comparative : on constate une amélioration de l'état des ongles ayant reçus la composition comprenant de l'acide silicique colloïdal. Les ongles sont plus solides et plus résistants, ils sont moins cassants et se dédoublent moins.

Exemple 3

On prépare un vernis à ongles ayant la composition suivante (% en poids) :

| | |
|---|---|
| . nitrocellulose | 10% |
| . plastifiant et résine | 15% |
| . acide silicique colloïdal (commercialisé sous la marque AEROSIL 200 de Degussa) | 1,5% |
| . particules de nacre | 0,2% |
| . pigment | 0,5% |
| . actifs, colorants et additifs | 1% |
| . solvant (acétates d'éthyle et de butyle) | qsp 100% |

On obtient un vernis qui s'applique aisément sur l'ongle. Il permet, après séchage, l'obtention d'un film lisse et homogène.

Test dévaluation

Le vernis de l'exemple 1 a fait l'objet d'un test d'évaluation sur un panel de femmes, qui ont été séparées en deux catégories : celles ayant des ongles mous (première catégorie) et celles ayant des ongles durs (seconde catégorie).

Ces femmes ont appliqué cette composition pendant 8 semaines et ont évalué (notation de 1 à 5) après 4 semaines ($N_1$) et après 8 semaines de traitement ($N_2$) la dureté, la brillance, l'aspect lisse, le caractère cassant, le dédoublement et l'état général de leurs ongles.

La même évaluation ($N_0$) avait été faite avant le début du traitement.

Une moyenne a été faite de ces évaluations, qui a ensuite été convertie en pourcentage d'amélioration de la note

moyenne après quatre ($M_1$) et après huit semaines ($M_2$) par rapport à la note moyenne après traitement :

$$M_i=(N_i-N_0)/N_0$$

i=1,2

On obtient les résultats suivants:

.   sur les femmes ayant au début de l'étude des ongles mous (17 cas) :

|  |  | M1 | M2 |
|---|---|---|---|
| Dureté | amélioration de la dureté | 55% | 50% |
| Ongles cassants | réduction du caractère cassant | 28% | 38% |
| Dédoublement | réduction du dédoublement | 31% | 31% |
| Etat général | amélioration | 41% | 36% |

.   sur les femmes ayant au début de l'étude des ongles durs (13 cas) :

|  |  | M1 | M2 |
|---|---|---|---|
| Brillance | amélioration de la brillance | 20% | 28% |
| Aspect lisse | amélioration | 45% | 60% |
| Ongles cassants | réduction du caractère cassant | 40% | 54% |
| Dédoublement | réduction du dédoublement | 37% | 50% |
| Etat général | amélioration | 52% | 52% |

On constate que l'ensemble de ces améliorations sont significatives dès 1 mois puis se stabilisent.

Exemple 4

On prépare un vernis à ongles ayant la composition suivante (% en poids) :

| . dispersion de polymère acrylique (40% de matière sèche) | 38% |
|---|---|
| . dispersion de polyuréthanne (30% de matière sèche) | 50% |
| . acide silicique colloïdal (commercialisé sous la marque Aerosil MOX80) | 0,7% |
| . additifs (colorants, actifs) | 1,3% |
| . eau | qsp 100% |

On obtient un vernis qui s'applique aisément sur l'ongle, et qui permet, après séchage, d'obtenir un film cosmétiquement acceptable.

Exemple 5

On compare le vernis de l'exemple 4 avec un vernis témoin de composition :

| . dispersion de polymère acrylique (40% de MS) | 38% |
|---|---|
| . dispersion de polyuréthanne (30% de MS) | 50% |
| . additifs (colorants, actifs) | 1,3% |
| . eau | qsp 100% |

On applique ces deux compositions pendant 8 semaines sur des ongles fragilisés (striés, fendus, mous ou souples, et ayant tendance à se dédoubler).
On applique le vernis selon l'exemple 4 sur tous les ongles d'une seule main, le vernis témoin étant appliquée sur l'autre main.
On observe les résultats suivants, obtenus par auto-évaluation comparative : on constate une amélioration de l'état des ongles ayant reçus le vernis comprenant de l'acide silicique colloïdal. Les ongles sont plus solides et plus résistants, ils sont moins cassants et se dédoublent moins.

Exemple 6

On prépare une base de soin pour ongles ayant la composition suivante :

| . huile végétale | 5 % |
|---|---|
| . acide silicique colloïdal | 1 % |
| . huile de silicone volatile | 94 % |

On obtient une huile de soin pour ongles qui dépose un film protecteur de l'ongle, qui pénètre par massage au niveau de la matrice et de la tablette unguéale.

Exemple 7

On compare la base de soin selon l'exemple 6, avec une base témoin ayant la composition suivante :

| . huile végétale | 5 % |
|---|---|
| . huile de silicone volatile | 95 % |

On applique ces compositions pendant 8 semaines sur des ongles fragilisés (striés, fendus, mous ou souples, et ayant tendance à se dédoubler).
On applique la base de l'ex. 6 sur tous les ongles d'une seule main, la base témoin étant appliquée sur l'autre main. On observe les résultats suivants, obtenus par auto-évaluation comparative : on constate une amélioration de l'état des ongles ayant reçus la base comprenant de l'acide silicique colloïdal. Les ongles sont plus solides et plus résistants, ils sont moins cassants et se dédoublent moins.

Exemple 8

On prépare une base de soin pour ongles ayant la composition suivante :

| . huile végétale | 4 % |
|---|---|
| . huile minérale | 1% |
| . acide silicique colloïdal | 0,5 % |
| . additifs (actifs et colorants) | 1,5% |
| . solvant | 1% |
| . huile de silicone volatile | 92 % |

On obtient une huile de soin pour ongles qui dépose un film protecteur de l'ongle, qui pénètre par massage au niveau de la matrice et de la tablette unguéale.

**Revendications**

1. Utilisation de l'acide silicique colloïdal en tant qu'agent renforçateur des ongles, pour obtenir des ongles moins cassants, l'acide silicique colloïdal étant appliqué sur l'ongle.

2. Utilisation de l'acide silicique colloïdal en tant qu'agent renforçateur des ongles, pour obtenir des ongles qui ne se dédoublent pas, l'acide silicique colloïdal étant appliqué sur l'ongle.

3. Utilisation de l'acide silicique colloïdal en tant qu'agent renforçateur des ongles, pour obtenir des ongles qui ne se fendillent pas, l'acide silicique colloïdal étant appliqué sur l'ongle.

4. Utilisation de l'acide silicique colloïdal en tant qu'agent renforçateur des ongles, pour diminuer la fragilisation des ongles affaiblis, notamment des ongles striés, fendus, mous ou souples, l'acide silicique colloïdal étant appliqué sur l'ongle.

**5.** Utilisation selon l'une quelconque des revendications précédentes, dans laquelle l'acide silicique colloïdal est choisi parmi les silices traitées en surface, les silices pyrogénées hydrophiles et/ou les silices pyrogénées hydrophobes.

**6.** Utilisation selon l'une quelconque des revendications précédentes, dans une composition comprenant au moins une matière filmogène et au moins un solvant.

**7.** Utilisation selon l'une quelconque des revendications précédentes, dans laquelle l'acide silicique colloïdal est présent en une quantité de 0,1 % à 5 % en poids, de préférence 1 - 2 % en poids, par rapport au poids total de la composition.

**8.** Utilisation selon l'une des revendications 6 ou 7, **caractérisée en ce que** le solvant est organique.

**9.** Utilisation selon l'une des revendications 6 à 8, dans laquelle la matière filmogène est choisie parmi les résines alkydes, acryliques et/ou vinyliques, les polyuréthannes et les polyesters, les celluloses et dérivés cellulosiques telles que la nitrocellulose, et les résines résultant de la condensation de formaldéhyde avec une arylsulfonamide, et leurs mélanges.

**10.** Utilisation selon l'une des revendications 8 ou 9, dans laquelle le solvant organique est choisi parmi le toluène, le xylène, l'acétate d'éthyle et/ou de butyle, les cétones, les éthers de glycol, les esters et les alcools tels que l'éthanol, l'isopropanol ou le butanol, et leurs mélanges.

**11.** Utilisation selon l'une des revendications 6 à 10, dans laquelle la composition cosmétique comprend en outre un agent plastifiant choisi parmi les citrates, les phtalates, les esters et/ou le camphre, généralement en une quantité de 5-30% en poids par rapport au poids de la composition.

**12.** Utilisation selon la revendication 6, **caractérisée en ce que** le solvant est de l'eau dans laquelle des particules de polymère filmogène sont en dispersion.

**13.** Utilisation selon la revendication 12, dans laquelle le polymère filmogène est choisi parmi les polyuréthannes, les polyester-polyuréthannes, les polyétherpolyuréthannes, les polymères radicalaires notamment de type acrylique, acrylique styrène et/ou vinylique, les polyesters, les résines alkydes, seuls ou en mélange.

**14.** Utilisation selon l'une des revendications précédentes, dans une composition se présentant sous la forme d'un produit à appliquer sur les ongles, tel qu'un vernis, une base ou une base de soin.

**15.** Utilisation selon l'une des revendications 1 à 5, dans une composition comprenant un milieu huileux.

**16.** Utilisation selon la revendication 15, dans laquelle l'acide silicique colloïdal est présent en une quantité de 0,01 à 10% en poids, de préférence 0,3-3% en poids, par rapport au poids total de la composition.

**17.** Utilisation selon l'une des revendications 15 à 16, dans laquelle le milieu huileux comprend une ou plusieurs huiles choisies parmi les huiles d'origine végétale, minérale, animale et/ou de synthèse, volatiles et/ou non volatiles.

**18.** Utilisation selon l'une des revendications 15 à 17, dans laquelle le milieu huileux comprend une ou plusieurs huiles choisies parmi les esters d'acide gras et de polyols ; les huiles de tournesol, de maïs, de soja, de courge, de pépins de raisin, de sésame, de noisette, d'abricot, d'amandes, d'avocat ; les huiles de poisson ; le tricaprocaprylate de glycérol ; les huiles végétales ou animales de formule $R_9COOR_{10}$ dans laquelle $R_9$ représente le reste d'un acide gras supérieur comportant de 7 à 19 atomes de carbone et $R_{10}$ représente une chaîne hydrocarbonée ramifiée contenant de 3 à 20 atomes de carbone, par exemple, l'huile de Purcellin ; les huiles essentielles naturelles ou synthétiques telles que les huiles d'eucalyptus, de lavandin, de lavande, de vétiver, de citron, de santal, de romarin, de camomille, de sarriette, de noix de muscade, de cannelle, d'hysope, de carvi, d'orange, de géraniol, de cade et de bergamote ; les hydrocarbures, tels que l'hexadécane et l'huile de paraffine ; les esters d'acide minéral et d'un alcool ; les éthers et les polyéthers ; les huiles de silicones.

**19.** Utilisation selon l'une des revendications 15 à 18, dans laquelle le milieu huileux comprend en outre une phase aqueuse, avec laquelle il peut former une solution ou une émulsion huile-dans-eau, eau-dans-huile, voire une émulsion multiple.

20. Utilisation selon l'une des revendications 15 à 19, dans une composition se présentant sous la forme d'une base huileuse ou d'une émulsion, telle qu'une base de soin pour ongles.


**Claims**

1. Use of colloidal silicic acid as an agent for strengthening the nails, in order to obtain nails which are less brittle, the colloidal silicic acid being applied to the nail.

2. Use of colloidal silicic acid as an agent for strengthening the nails, in order to obtain nails which do not split, the colloidal silicic acid being applied to the nail.

3. Use of colloidal silicic acid as an agent for strengthening the nails, in order to obtain nails which do not crack, the colloidal silicic acid being applied to the nail.

4. Use of colloidal silicic acid as an agent for strengthening the nails, in order to reduce the fragility of weakened nails, in particular striated, cracked, soft or flexible nails, the colloidal silicic acid being applied to the nail.

5. Use according to any one of the preceding claims, in which the colloidal silicic acid is chosen from surface-treated silicas, hydrophilic pyrogenic silicas and/or hydrophobic pyrogenic silicas.

6. Use according to any one of the preceding claims, in a composition comprising at least one film-forming substance and at least one solvent.

7. Use according to any one of the preceding claims, in which the colloidal silicic acid is present in an amount of from 0.1% to 5% by weight, preferably 1 - 2% by weight, relative to the total weight of the composition.

8. Use according to either of Claims 6 and 7, **characterized in that** the solvent is organic.

9. Use according to one of Claims 6 to 8, in which the film-forming substance is chosen from alkyd, acrylic and/or vinyl resins, polyurethanes and polyesters, celluloses and cellulose derivatives such as nitrocellulose, and resins resulting from the condensation of formaldehyde with an arylsulphonamide, and mixtures thereof.

10. Use according to either of Claims 8 and 9, in which the organic solvent is chosen from toluene, xylene, ethyl acetate and/or butyl acetate, ketones, glycol ethers, esters and alcohols such as ethanol, isopropanol or butanol, and mixtures thereof.

11. Use according to one of Claims 6 to 10, in which the cosmetic composition also comprises a plasticizer chosen from citrates, phthalates, esters and/or camphor, generally in an amount of 5 - 30% by weight relative to the weight of the composition.

12. Use according to Claim 6, **characterized in that** the solvent is water in which film-forming polymer particles are dispersed.

13. Use according to Claim 12, in which the film-forming polymer is chosen from polyurethanes, polyester polyurethanes, polyether polyurethanes, radical polymers in particular of the acrylic, styrene acrylic and/or vinylic type, polyesters and alkyd resins, alone or as a mixture.

14. Use according to one of the preceding claims, in a composition in the form of a product to be applied to the nails, such as a varnish, a base or a care base.

15. Use according to one of Claims 1 to 5, in a composition comprising an oily medium.

16. Use according to Claim 15, in which the colloidal silicic acid is present in an amount of from 0.01 to 10% by weight, preferably 0.3 - 3% by weight, relative to the total weight of the composition.

17. Use according to either of Claims 15 and 16, in which the oily medium comprises one or more oils chosen from volatile and/or non-volatile oils of plant, mineral, animal and/or synthetic origin.

18. Use according to one of Claims 15 to 17, in which the oily medium comprises one or more oils chosen from fatty acid esters of polyols; sunflower oil, corn oil, soybean oil, marrow oil, grapeseed oil, sesame oil, hazelnut oil, apricot oil, almond oil, avocado oil; fish oils; glyceryl tricaprocaprylate; plant or animal oils of formula $R_9COO_{10}$ in which $R_9$ represents a higher fatty acid residue containing from 7 to 19 carbon atoms and $R_{10}$ represents a branched hydrocarbon chain containing from 3 to 20 carbon atoms, for example purcellin oil; natural or synthetic essential oils such as eucalyptus oil, hybrid lavender oil, lavender oil, vetiver oil, lemon oil, sandalwood oil, rosemary oil, camomile oil, savory oil, nutmeg oil, cinnamon oil, hyssop oil, caraway oil, orange oil, geraniol oil, cade oil and bergamot oil; hydrocarbons such as hexadecane and liquid paraffin; inorganic acid esters of an alcohol; ethers and polyethers; silicone oils.

19. Use according to one of Claims 15 to 18, in which the oily medium also comprises an aqueous phase in which it can form a solution or an oil-in-water or water-in-oil emulsion or even a multiple emulsion.

20. Use according to one of Claims 15 to 19, in a composition in the form of an oily base or an emulsion, such as a nailcare base.

**Patentansprüche**

1. Verwendung von kolloidaler Kieselsäure als Mittel zur Stärkung der Nägel, um Nägel zu erhalten, die weniger bruchempfindlich sind, wobei die kolloidale Kieselsäure auf den Nagel aufgetragen wird.

2. Verwendung von kolloidaler Kieselsäure als Mittel zur Stärkung der Nägel, um Nägel zu erhalten, die nicht einreißen oder sich nicht spalten, wobei die kolloidale Kieselsäure auf den Nagel aufgetragen wird.

3. Verwendung von kolloidaler Kieselsäure als Mittel zur Stärkung der Nägel, um Nägel zu erhalten, die keine Risse bekommen, wobei die kolloidale Kieselsäure auf den Nagel aufgetragen wird.

4. Verwendung von kolloidaler Kieselsäure als Mittel zur Stärkung der Nägel, um die Schwächung geschwächter Nägel, insbesondere von geriffelten, gespaltenen bzw. abblätternden, dünnen, weichen oder biegbaren Nägeln, zu vermindern, wobei die kolloidale Kieselsäure auf den Nagel aufgetragen wird.

5. Verwendung nach einem der vorhergehenden Ansprüche, wobei die kolloidale Kieselsäure unter Kieselsäuren, die an der Oberfläche behandelt sind, hydrophilen pyrogenen Kieselsäuren und/oder hydrophoben pyrogenen Kieselsäuren ausgewählt wird.

6. Verwendung nach einem der vorhergehenden Ansprüche in einer Zusammensetzung, die mindestens ein filmbildendes Material und mindestens ein Lösemittel enthält.

7. Verwendung nach einem der vorhergehenden Ansprüche, wobei die kolloidale Kieselsäure in einer Menge von 0,1 bis 5 Gew.-%, vorzugsweise 1 bis 2 Gew.-%, bezogen auf das Gesamtgewicht der Zusammensetzung, enthalten ist.

8. Verwendung nach einem der Ansprüche 6 und 7, **dadurch gekennzeichnet, daß** das Lösemittel ein organisches Lösemittel ist.

9. Verwendung nach einem der Ansprüche 6 bis 8, wobei das filmbildende Material unter den Alkyd-, Acryl- und/oder Vinylharzen, den Polyurethanen und den Polyestern, den Cellulosen und Cellulosederivaten, wie Nitrocellulose, und den Harzen, die bei der Kondensation von Formaldehyd mit einem Arylsulfonamid entstehen, sowie deren Gemischen ausgewählt wird.

10. Verwendung nach einem der Ansprüche 8 und 9, wobei das organische Lösemittel unter Toluol, Xylol, Ethylacetat und/oder Butylacetat, den Ketonen, Glykolethern, Estern und Alkoholen, wie Ethanol, Isopropanol und Butanol, sowie deren Gemischen ausgewählt wird.

**11.** Verwendung nach einem der Ansprüche 6 bis 10, wobei die kosmetische Zusammensetzung außerdem einen Weichmacher, der unter den Citraten, den Phthalaten, den Estern und/oder Campher ausgewählt wird, im allgemeinen in einer Menge von 5 bis 30 Gew.-%, bezogen auf das Gewicht der Zusammensetzung, enthält.

**12.** Verwendung nach Anspruch 6, **dadurch gekennzeichnet, daß** es sich bei dem Lösemittel um Wasser handelt, in dem filmbildende Polymerpartikel dispergiert sind.

**13.** Verwendung nach Anspruch 12, wobei das filmbildende Polymer unter den Polyurethanen, den Polyesterpolyurethanen, den Polyetherpolyurethanen, den radikalischen Polymeren, insbesondere vom Acryl-, Styrolacryl- und/oder Vinyl-Typ, den Polyestern, den Alkydharzen, einzeln oder im Gemisch, ausgewählt wird.

**14.** Verwendung nach einem der vorhergehenden Ansprüche in einer Zusammensetzung, die in Form eines Produkts, das auf die Nägel aufgetragen wird, wie z.B. eines Nagellacks, einer Basisformulierung oder einer pflegenden Basisformulierung, vorliegt.

**15.** Verwendung nach einem der Ansprüche 1 bis 5 in einer Zusammensetzung, die ein öliges Medium umfaßt.

**16.** Verwendung nach Anspruch 15, wobei die kolloidale Kieselsäure in einer Menge von 0,01 bis 10 Gew.-%, vorzugsweise 0,3 bis 3 Gew.-%, bezogen auf das Gesamtgewicht der Zusammensetzung, enthalten ist.

**17.** Verwendung nach einem der Ansprüche 15 und 16, wobei das ölige Medium ein oder mehrere Öle enthält, die unter Ölen pflanzlicher, anorganischer, tierischer und/oder synthetischer Herkunft, die flüchtig oder nicht flüchtig sind, ausgewählt werden.

**18.** Verwendung nach einem der Ansprüche 15 bis 17, wobei das ölige Medium ein oder mehrere Öle enthält, die ausgewählt werden unter den Estern aus einer Fettsäure und Polyolen, Sonnenblumenöl, Maisöl, Sojaöl, Kürbisöl, Traubenkernöl, Sesamöl, Haselnußöl, Aprikosenöl, Mandelöl und Avocadoöl, Fischölen, Glycerintricaprocaprylat, pflanzlichen oder tierischen Ölen der Formel $R_9COOR_{10}$, worin $R_9$ den Rest einer höheren Fettsäure mit 7 bis 19 Kohlenstoffatomen und $R_{10}$ eine verzweigte Kohlenwasserstoffkette mit 3 bis 20 Kohlenstoffatomen darstellt, beispielsweise Purcellinöl, natürlichen und synthetischen etherischen Ölen, wie z.B. Eukalyptusöl, Lavandinöl, Lavendelöl, Vetiveröl, Zitronenöl, Sandelholzöl, Rosmarinöl, Kamillenöl, Bergminzeöl, Muskatnußöl, Zimtöl, Ysopöl, Kümmelöl, Orangenöl, Geranienöl, Wacholderteeröl, Bergamotteöl, Kohlenwasserstoffen, wie z.B. Hexadecan und Paraffinöl, Estern aus einer anorganischen Säure und einem Alkohol, Ethern und Polyethern, Siliconölen.

**19.** Verwendung nach einem der Ansprüche 15 bis 18, wobei das ölige Medium außerdem eine wäßrige Phase umfaßt, mit der es eine Lösung, eine Öl-in-Wasser-Emulsion, eine Wasser-in-Öl-Emulsion oder auch eine multiple Emulsion bilden kann.

**20.** Verwendung nach einem der Ansprüche 15 bis 19 in einer Zusammensetzung, die in Form einer öligen Basisformulierung oder einer Emulsion, wie z.B. einer die Nägel pflegenden Basisformulierung, vorliegt.